(19) Europäisches Patentamt
European Patent Office
Office européen des brevets

(11) Veröffentlichungsnummer: **0 120 465**
**B1**

(12) # EUROPÄISCHE PATENTSCHRIFT

(45) Veröffentlichungstag der Patentschrift: **23.07.86**

(51) Int. Cl.⁴: **C 07 D 295/18**, C 07 D 213/72, C 07 D 239/42, C 07 D 241/20

(21) Anmeldenummer: **84103136.2**

(22) Anmeldetag: **22.03.84**

(54) **Neue N-Phenyl-N'-Cycloalkylalkanoyl-piperazine und Verfahren zu deren Herstellung.**

(30) Priorität: **25.03.83 DE 3310871**

(43) Veröffentlichungstag der Anmeldung: **03.10.84 Patentblatt 84/40**

(45) Bekanntmachung des Hinweises auf die Patenterteilung: **23.07.86 Patentblatt 86/30**

(84) Benannte Vertragsstaaten: **AT BE CH DE FR GB IT LI LU NL SE**

(56) Entgegenhaltungen:
**CH-A-619 462**
**DE-A-2 623 772**
**DE-B-1 695 328**
**DE-B-2 739 659**

**JOURNAL OF MEDICINAL CHEMISTRY, Band 11, 1968, Easton TSUTOMU IRIKURA et al. "New Analgetic Agents. V. 1-Butyryl-4-cinnanylpiperazine Hydrochloride and Related Compounds" Seiten 801-804**
**JOURNAL OF MEDICINAL CHEMISTRY, Band 12, 1969, Easton R.B. PETIGARA et al. "Synthesis and Central Nervous System Depressant Activity of New Piperazine and Related Derivatives. III." Seiten 865-870**

(73) Patentinhaber: **Degussa Aktiengesellschaft, Weissfrauenstrasse 9, D-6000 Frankfurt am Main 1 (DE)**

(72) Erfinder: **Oepen, Gerhard, Dr., Fichtelgebirgsstrasse 129, D-6450 Hanau 6 (DE)**
Erfinder: **Engel, Jürgen, Dr., Cranachstrasse 25, D-8755 Alzenau (DE)**
Erfinder: **Jakovlev, Vladimir, Dr., Herm. Löns-Strasse 7, D-6457 Maintal 1 (DE)**
Erfinder: **Thiemer, Klaus, Dr., Fürstenbergstrasse 24, D-6450 Hanau 9 (DE)**

**EP 0 120 465 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Piperazin-Verbindungen der Formel

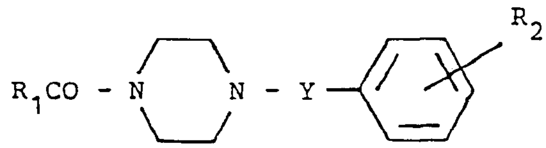

worin $R_2$ Wasserstoff oder ein Chloratom in 2-Stellung und $R_1$ eine niedere Alkylgruppe, ein Phenylrest, ein Benzylrest oder ein Chlorphenylrest ist, falls Y eine einfache Bindung zwischen dem Piperazinring und dem Phenylrest darstellt, oder worin $R_1$ eine niedere Alkylgruppe, ein Phenylrest, ein Benzylrest, ein Chlorphenylrest, oder ein Methoxyphenylrest ist, falls Y Methylen, Dimethylen, Trimethylen oder die Gruppe $—CH_2—CH=CH—$ ist, sind bekannt. Einige dieser Verbindungen, insbesondere solche, wo Y die Gruppe $—CH_2—CH=CH—$ ist, erwiesen sich als analgetisch wirksam (J. Med. Chem. 11, Seite 803 (1968)).

Weiterhin sind Piperazin-Verbindungen der folgenden Formel

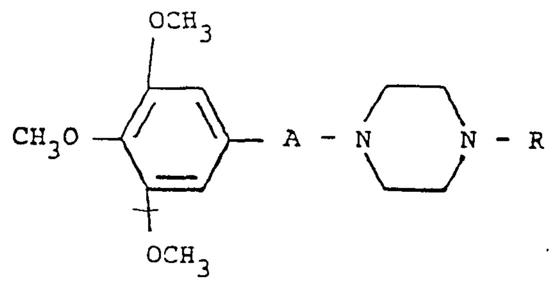

bekannt. In dieser Formel bedeutet A beispielsweise die Gruppe $—CH_2—CH_2—CO—$ und R einen 2-Chlorphenylrest, einen 2-Methyl-phenylrest oder einen Pyridyl-(2)-rest. Diese Verbindungen wurden untersucht, ob sie eine dämpfende Wirkung auf das Zentralnervensystem (sedative und ataktische Wirkungen; tranquillierende Wirkung) besitzen, wobei einige eine Wirkung zeigten (J. Med. Chem. 12, Seite 867 (1969)).

Schließlich werden in der deutschen Offenlegungsschrift 26 23 772 Piperazin-Derivate der allgemeinen Formel

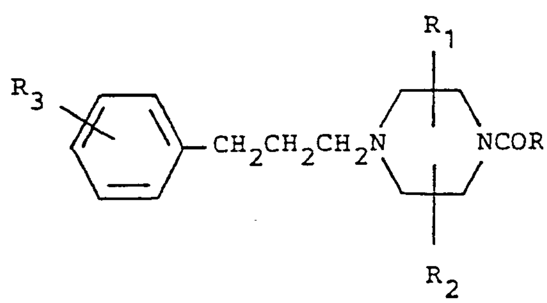

(I)

beschrieben, in der R eine niedrigmolekulare, geradkettige oder verzweigte Alkylgruppe mit 1 bis 6 Kohlenstoffatomen, eine niedrigmolekulare, geradkettige oder verzweigte, ungesättigte Alkylgruppe mit 2 bis 5 Kohlenstoffatomen, eine Cycloalkylgruppe mit 3 bis 6 Kohlenstoffatomen, eine niedrigmolekulare, geradkettige oder verzweigte Alkylgruppe mit 1 bis 6 Kohlenstoffatomen, die mit einer niedrigmolekularen, geradkettigen oder verzweigten Alkoxygruppe mit 1 bis 6 Kohlenstoffatomen oder einer Hydroxygruppe verbunden ist, eine niedrigmolekulare, geradkettige oder verzweigte, ungesättigte Alkylgruppe mit 2 bis 5 Kohlenstoffatomen, die mit einer niedrigmolekularen, geradkettigen oder verzweigten Alkoxygruppe mit 1 bis 6 Kohlenstoffatomen verbunden ist, oder eine Tetrahydrofurangruppe; $R_1$ und $R_2$, die gleichartig oder verschieden sein können, Wasserstoffatome oder niedrigmolekulare, geradkettige oder verzweigte Alkylgruppen mit 1 bis 4 Kohlenstoffatomen (mit der Maßgabe, daß nicht beide Gruppen $R_1$ und $R_2$ ein Wasserstoffatom bedeuten); und $R_3$ ein Wasserstoffatom, ein Halogenatom, eine niedrigmolekulare, geradkettige oder verzweigte Alkylgruppe mit 1 bis 4 Kohlenstoffatomen, eine niedrigmolekulare, geradkettige oder verzweigte Alkoxygruppe mit 1 bis 4 Kohlenstoffatomen oder eine Hydroxygruppe bedeuten.

Für diese Verbindungen wird eine analgetische Wirkung angegeben, wobei es sich hierbei jedoch um peripher wirksame Substanzen handelt. Demgegenüber stellen die erfindungsgemäßen Verbindungen zentral wirksame Analgetika dar.

Die Erfindung betrifft die durch die Patentansprüche definierten Gegenstände.

Die erfindungsgemäßen Verbindungen sind pharmakologisch beziehungsweise pharmakotherapeutisch wirksam. Beispielsweise sind sie analgetisch wirksam. Sie besitzen eine hohe

therapeutische Breite und sind weiterhin durch das Fehlen von zentralnervösen Nebenwirkungen wie zum Beispiel Sedierung und Ataxie, gekennzeichnet. Die erfindungsgemäßen Verbindungen stellen zentral wirksame Analgetika dar. Toleranz-Versuche zeigten, daß die erfindungsgemäßen Verbindungen zu keiner Gewöhnung (Sucht) führen. Die analgetische Wirkung ist nicht opiatartig, das heißt die erfindungsgemäßen Verbindungen zeigen keine Affinität zum Opiatrezeptor (beispielsweise keine Antagonisierung der Analgesie bei nachfolgender Gabe von Naloxon). Darüberhinaus hemmen die erfindungsgemäßen Verbindungen die Magensäuresekretion und sind antiulcerativ und antiphlogistisch wirksam.

Der Erfindung liegt also die Aufgabe zugrunde, Verbindungen mit günstigen pharmakologischen Eigenschaften, die als Arzneimittel verwertbar sind, zur Verfügung zu stellen.

Die in den Verbindungen der Formel I auftretenden Alkylreste (beispielsweise als Alkylgruppe oder in Form der Alkoxygruppe, der Alkylmercaptogruppe, der Alkylaminogruppe, der Dialkylaminogruppe oder der Phenalkoxygruppe) und Alkenyloxyreste sowie die Alkylenkette alk können gerade oder verzweigt sein. Falls die Reste $R_3$ und/oder $R_4$ Alkylgruppen sind oder Alkylreste enthalten, bestehen diese Alkylreste insbesondere aus 1 bis 4 Kohlenstoffatomen (Methyl-, Äthyl-, Propyl-, Isopropyl-, Butylgruppe). Die Kette alk bedeutet eine Alkylengruppe wie zum Beispiel die Methylen-, Dimethylen-, Trimethylen-, Tetramethylen-, Pentamethylen- oder Hexamethylengruppe oder auch zum Beispiel die Gruppe

$$-\underset{\displaystyle CH_3}{\underset{|}{CH}}-CH_2-,$$

$$-\underset{\displaystyle CH_3}{\underset{|}{CH}}-(CH_2)_2- \quad \text{oder}$$

$$-CH_2-\underset{\displaystyle CH_3}{\underset{|}{CH}}-CH_2-;$$

insbesondere besteht die Kette alk aus 1, 2 oder 3 C-Atomen. Falls der Rest $R_3$ oder $R_4$ eine $C_3$—$C_6$-Alkenyloxygruppe ist, besteht der Alkenylteil insbesondere aus 3 oder 4 C-Atomen. Falls der Rest $R_2$ einen $C_3$—$C_{16}$-Cycloalkylrest darstellt, handelt es sich beispielsweise um die Cyclopropyl-, Cyclobutyl-, Cyclopentyl-, Cyclohexyl-, Cycloheptyl-, Cyclononyl-, Cyclooctyl- oder Cyclodecylgruppe. Falls die Reste $R_3$ oder $R_4$ $C_3$—$C_6$-Cycloalkyloxygruppen bedeuten, handelt es sich um Cyclopropyloxy-, Cyclobutyloxy-, Cyclopentyloxy- oder Cyclohexyloxygruppen.

Falls die Reste $R_3$ oder $R_4$ Alkanoyloxygruppen, Alkanoylaminogruppen oder Alkanoylgruppen bedeuten, besteht der Alkanoylrest insbesondere aus 2 bis 4 C-Atomen (zum Beispiel Acetylrest, Propionylrest, Butyrylrest).

Der Pyridylrest ($R_1$) ist vorzugsweise über die 2-Stellung mit dem Piperazinrest verknüpft. Das gleiche gilt hinsichtlich des Pyrimidyl- und Pyrazinylrestes. Die Reste $R_3$ und $R_4$ können an der 2-, 3- und/oder 4-Stellung des Phenylrestes $R_1$ sitzen. Falls dieser Phenylrest einen Substituenten enthält, befindet sich dieser vorzugsweise in 2-Stellung, falls er 2 Substituenten $R_3$ und $R_4$ enthält, befinden sich diese vorzugsweise in 2,6-Stellung des Phenylrestes. Besonders günstige Wirkungen zeigen Verbindungen I, worin einer oder beide Substituenten $R_3$ und/oder $R_4$ $C_1$—$C_6$-Alkoxygruppen, insbesondere $C_1$—$C_4$-Alkoxygruppen, $C_2$—$C_6$-Alkanoylaminogruppen, insbesondere $C_2$—$C_4$-Alkanoylaminogruppen oder Fluor oder Chlor bedeuten, alk 2 oder 3 C-Atome enthält und $R_2$ ein Cyclohexylrest ist. Dabei können $R_3$ und $R_4$ gleiche oder verschiedene der genannten Substituenten enthalten.

Falls $R_1$ ein Pyridylrest oder ein Pyrazinylrest darstellt, und dieser einen Substituenten $R_3$ enthält, befindet sich jener vorzugsweise in der 6-Stellung des Pyridylbeziehungsweise Pyrazinylrestes. Bei 2 Substituenten ist der Pyridylrest vorzugsweise in 3- und 6-Stellung, der Pyrazinylrest vorzugsweise in 5- und 6-Stellung durch die Substituenten $R_3$ und $R_4$ substituiert.

Die Verfahrensprodukte können gegebenenfalls alkyliert beziehungsweise alkenyliert werden. Hierbei handelt es sich beispielsweise um die Einführung einer $C_1$—$C_6$-Alkylgruppe, einer $C_3$—$C_6$-Cycloalkylgruppe, einer Phenyl-$C_1$—$C_4$-alkylgruppe beziehungsweise einer $C_3$—$C_6$-Alkenylgruppe in Verbindungen, worin $R_3$ und/oder $R_4$ eine Amino- oder Monoalkylaminogruppe oder eine Hydroxygruppe bedeuten.

Diese Alkylierungen erfolgen in an sich bekannter Weise. Als Alkylierungsmittel kommen beispielsweise in Betracht: Ester der Formel R'Hal, $ArSO_2OR'$ und $SO_2(OR')_2$, wobei Hal ein Halogenatom (insbesondere Chlor, Brom oder Jod) und Ar ein aromatischer Rest wie zum Beispiel ein gegebenenfalls durch einen oder mehrere niedere Alkylreste substituierter Phenyl- oder Naphthylrest ist und R' eine $C_1$—$C_6$-Alkylgruppe, eine $C_3$—$C_6$-Alkenylgruppe, eine $C_3$—$C_6$-Cycloalkylgruppe oder eine Phenyl-$C_1$—$C_4$-alkylgruppe ist. Beispiele sind p-Toluolsulfonsäure-$C_1$—$C_6$-alkylester, p-Toluolsulfonsäure-$C_3$—$C_6$-alkenylester, $C_1$—$C_6$-Dialkylsulfate, $C_1$—$C_6$-Alkylhalogenide, $C_3$—$C_6$-Alkenylhalogenide, $C_3$—$C_6$-Cycloalkylhalogenide, Phenyl-$C_1$—$C_4$-alkylhalogenide und ähnliche. Die Alkylierungsreaktion wird gegebenenfalls unter Zusatz von üblichen säurebindenden Mitteln wie Alkalicarbonaten ($K_2CO_3$), Alkalihydroxiden (NaOH,

KOH), Pyridin oder anderen üblichen tertiären Aminen, bei Temperaturen zwischen 0 und 200°C, vorzugsweise 20 bis 150°C in inerten Lösungsmitteln wie niederen Alkoholen (Methanol, Ethanol, Isopropanol), niederen Ketonen (Aceton, Methylethylketon), niederen Halogenalkanen (Chloroform, Methylenchlorid, Dichlorethan), Dioxan, Dimethylformamid, Dimethylsulfoxyd, aromatischen Kohlenwasserstoffen (Benzol, Toluol, Xylol) oder Pyridin vorgenommen.

Man kann bei dieser Alkylierung aber auch so vorgehen, daß man zuerst von der zu alkylierenden Verbindung der Formel I, worin beispielsweise $R_3$ und/oder $R_4$ eine Amino-, Monoalkylamino- oder Hydroxygruppe ist, eine Alkaliverbindung herstellt, indem man sie in einem inerten Lösungsmittel wie Dioxan, Tetrahydrofuran, Dimethylformamid, Benzol, Toluol oder Xylol oder in flüssigem Ammoniak mit einem Alkalimetall, Alkalihydrid oder Alkaliamid (insbesondere Natrium- oder Natriumverbindungen) bei Temperaturen zwischen −70 und 120°C umsetzt und dann das alkylierende Agens (zum Beispiel $C_1$—$C_6$-Alkyljodid oder $C_1$—$C_6$-Alkylbromid) bei einer Temperatur zwischen −70 und +50°C zufügt.

Die Alkylierung kann auch in Gegenwart von Tetraalkylammoniumsalzen (insbesondere der Halogenide) in Kombination mit Alkalihydroxiden bei Temperaturen zwischen 0—100°C, vorzugsweise 20—80°C in einem aprotischen Lösungsmittel oder auch in Chloroform oder Methylenchlorid vorgenommen werden. Als aprotische Lösungsmittel kommen zum Beispiel in Betracht: tertiäre Amide (Dimethylformamid, N-Methyl-pyrrolidon, Hexamethylphosphorsäuretriamid), Dimethylsulfoxid, Acetonitril, Dimethoxyethan, Aceton, Tetrahydrofuran.

In solche Verfahrensprodukte der Formel I, worin $R_3$ und/oder $R_4$ eine Aminogruppe oder eine Hydroxygruppe bedeuten, kann in die Amino- oder Hydroxygruppe durch Acylierung eine $C_2$—$C_6$-Alkanoylgruppe eingeführt werden. Diese Acylierung erfolgt beispielsweise in hierfür bekannter Weise unter Verwendung von $C_2$—$C_6$-Alkanoylhalogeniden, $C_2$—$C_6$-Alkanoylanhydriden. Beispielsweise erfolgt diese Acylierung in einem Lösungs- oder Suspensionsmittel (aliphatische Halogenkohlenwasserstoffe wie Chloroform, Dichlormethan, niedere aliphatische Ketone, Dioxan, Dimethylformamid, Benzol, Toluol) in Gegenwart eines säurebindenden Stoffes (Pyridin, Trialkylamine, Alkalicarbonate, Alkalihydrogencarbonate, Erdalkalicarbonate, Alkaliacetate) bei Temperaturen zwischen 0—180°C, vorzugsweise 0—100°C. Gegebenenfalls kann man bei der Acylierung auch so vorgehen, daß man zuerst von der zu acylierenden Verbindung eine Alkaliverbindung herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) bei Temperaturen zwischen 0 und 150°C umsetzt und dann das acylierende Agens (zum Beispiel das Alkanoylhalogenid) zufügt.

Anstelle der angeführten Acylierungsmittel können auch andere in der Chemie gebräuchliche chemisch äquivalente Mittel verwendet werden (siehe zum Beispiel auch L. F. und Mary Fieser "Reagents for Organic Synthesis", John Wiley and Sons, Inc., New York, 1967, Vol. 1, Seiten 1303—1304 und Vol. 2, Seite 471). Selbstverständlich können in den erhaltenen Verbindungen vorhandene Acylgruppen in bekannter Weise auch wieder abgespalten werden, beispielsweise mit wässrigem Alkali oder alkoholischer Alkalilauge (zum Beispiel methanolische KOH) oder gegebenenfalls auch mittels Mineralsäuren wie Salzsäure der Schwefelsäure in alkoholischer oder wässrig-alkoholischer Lösung bei Temperaturen zwischen 20 und 100°C.

Falls in den Verfahrensprodukten die Reste $R_3$ und/oder $R_4$ Nitrogruppen bedeuten, können diese zu den entsprechenden Aminogruppen reduziert werden.

Für diese Reduktion kommt insbesondere die katalytische Hydrierung in Betracht. Als Katalysatoren kommen zum Beispiel in Frage: Raney-Nickel, Edelmetalle, wie Palladium und Platin sowie Verbindungen davon mit und ohne Träger wie beispielsweise Bariumsulfat, Calciumsulfat und so weiter. Es empfiehlt sich, die Hydrierung der Nitrogruppe bei Temperaturen zwischen 20 und 80°C und einem Druck von ungefähr 5—50 atü (ungefähr 5—50 bar Überdruck) in einem Lösungsmittel, beispielsweise Alkoholen, Dioxan, Tetrahydrofuran und so weiter vorzunehmen. Für die anschließende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden. Die Reduktion kann aber auch mit nascierendem Wasserstoff, beispielsweise Zink/Salzsäure, Zinn/Salzsäure, Eisen/Salzsäure oder mit Salzen des Schwefelwasserstoffs in Alkohol/Wasser bei etwa 70 bis etwa 120°C oder mit aktiviertem Aluminium in wasserhaltigem Äther bei 20 bis 40°C oder mit Zinn(II)-Chlorid/Salzsäure durchgeführt werden.

Zu dem Verfahren a):

Dieses Verfahren wird im allgemeinen in einem inerten Lösungs- oder Suspensionsmittel bei Temperaturen zwischen 0—250°C — insbesondere 5—180°C, vorzugsweise 20—150°C — durchgeführt. Als Lösungsmittel kommen beispielsweise in Betracht:

Gesättigte alicyclische und cyclische Ether (Dioxan, Tetrahydrofuran, niedere Dialkylether wie Diethylether, Diisopropylether), niedere Alkanole wie Ethanol, Isopropanol, Butanol, niedere aliphatische Ketone (Aceton, Methylethylketon), niedere aliphatische Kohlenwasserstoffe oder Halogenkohlenwasserstoffe (Methylenchlorid, Chloroform, 1,2-Dichlorethan), aromatische Kohlenwasserstoffe (Benzol, Toluol, Xylol), niedere Dialkylamide von niederen gesättigten aliphatischen Carbonsäuren (Dimethylformamid, Dimethylacetamid), Tetramethylharnstoff, N-Methylpyrolidon, Dimethylsulfoxid beziehungsweise Mischungen dieser Mittel.

Falls X ein Halogenatom bedeutet, handelt es sich insbesondere um Chlor, Brom oder Jod, vorzugsweise um Chlor oder Brom.

Im allgemeinen werden die Reaktionskomponenten in molaren Mengen umgesetzt. Gegebenenfalls kann es jedoch zweckmäßig sein, eine Reaktionskomponente in leichtem Überschuß einzusetzen. Insbesondere gilt dies, falls die Verbindung der Formel III ein Carbonsäureester $R_2$—alk—CO—OR darstellt, wobei R eine niedere Alkylgruppe ist. (In diesem Fall empfiehlt es sich, gegebenenfalls den bei der Reaktion entstehenden niederen Alkohol laufend zu entfernen.) Gegebenenfalls kann die Umsetzung auch in Gegenwart von basischen beziehungsweise säurebindenden Mitteln, wie Alkalicarbonaten (Pottasche, Soda), Alkalihydrogencarbonaten, Alkaliacetaten, Alkalihydroxyden oder tertiären Aminen (beispielsweise Triethylamin) durchgeführt werden. Letzteres gilt insbesondere, wenn Verbindungen der Formel III eingesetzt werden, worin X ein Halogenatom bedeutet.

Gegebenenfalls ist auch der Zusatz von Kondensationsmitteln wie Dicyclohexylcarbodiimid, Tetraäthylpyrophosphit, 5-(3'-Sulfonphenyl)-äthylisooxazol, Schwefligsäure-bis-alkylamiden (zum Beispiel $SO[N(CH_3)_2]_2$) oder N,N'-Carbonyl-diimidazol günstig (falls beispielsweise X = OH ist). Falls R einen substituierten Phenylrest darstellt, enthält dieser vorzugsweise einen oder zwei der angegebenen Substituenten, wobei sich diese vorzugsweise in 3- und/oder 4-Stellung des Phenylrestes befinden.

Nichtbekannte Ausgangsstoffe der Formel III, worin X ein Halogenatom (vorzugsweise Chlor oder Brom) und Z' die Gruppe —CO—alk—$R_2$ ist, können beispielsweise aus den entsprechenden Säuren durch Umsetzung mit Thionylchlorid oder Thionylbromid in bekannter Weise erhalten werden. Beispielhaft wird die Herstellung von 3-Cyclohexyl-propionsäurechlorid angegeben:

Zu 135,6 g (1,14 Mol) Thionylchlorid werden innerhalb von 45 Minuten 60 g (0,38 Mol) 3-Cyclohexylpropionsäure zugetropft. Nach beendetem Zutropfen wird der Ansatz weitere 2 Stunden bei 50°C gerührt. Anschliessend wird das überschüssige Thionylchlorid im Vakuum eingeengt und der verbleibende Rückstand ohne weitere Reinigung umgesetzt.

Aus den soeben angeführten Säurechloriden beziehungsweise Säurehalogeniden können solche Ausgangsstoffe III, worin X die Gruppe OR und Z' die Gruppe —CO—alk—$R_2$ ist, in üblicher Weise beispielsweise durch Umsetzung mit Verbindungen HOR beziehungsweise deren Metallsalzen (Alkalisalzen) hergestellt werden (siehe hierzu Organikum, Organisch Chemisches Grundpraktikum, VEB; Deutscher Verlag der Wissenschaften Berlin, 9. Auflage 1970, Seite 440 ff.). Nichtbekannte Ausgangsstoffe der Formel II, worin Z die Gruppe —CO—alk—$R_2$ ist, können zum Beispiel durch Umsetzung von Benzylpiperazin mit den entsprechenden Säurechloriden $R_2$—alk—COCl und anschliessender Debenzylierung hergestellt werden. Die Reaktion des Benzylpiperazins mit dem Säurechlorid wird zum Beispiel bei 0°C in Aceton als Lösungsmittel durchgeführt. Nach Abziehen des Lösungsmittels wird das so erhaltene Rohprodukt in Methanol aufgenommen, mit Pd/C versetzt und bei 5 bar und 40°C debenzyliert. Nach Abfiltrieren des Katalysators wird das Lösungsmittel im Vakuum abdestilliert. Das auf diese Weise dargestellte Produkt kann ohne weitere Aufarbeitung weiterverarbeitet werden.

Zu dem Verfahren b):

Dieses Verfahren wird vorzugsweise in einem polaren Lösungsmittel bei Temperaturen zwischen 40—200°C durchgeführt. Vorzugsweise wird in Gegenwart eines Akzeptors für die im Verlauf der Reaktion gebildete Halogenwasserstoffsäure gearbeitet. Als Säureacceptor kommen beispielsweise in Betracht: Alkalicarbonate ($K_2CO_3$, $Na_2CO_3$, $NaHCO_3$), tertiäre Amine wie Triethylamin, Pyridin, Alkaliacetate, Alkalihydroxide.

In der einen Ausgangsverbindung für dieses Verfahren bedeutet Hal vorzugsweise Chlor oder Brom.

Als Lösungsmittel können zum Beispiel verwendet werden: niedere Alkanole (Ethanol, Isopropanol, Butanol, Isoamylalkohol), niedere alicyclische oder cyclische Ether (Diethylether, Dioxan, Tetrahydrofuran), Diethylenglykoldi-$C_1$—$C_4$-alkylether (diethylenglykoldimethylether), niedere Dialkylamide von niederen gesättigten aliphatischen Carbonsäuren (Dimethylformamid, Dimethylacetamid), Tetramethylharnstoff, N-Methylpyrrolidon, Dimethylsulfoxid sowie Mischungen dieser Mittel.

Ausgangsstoffe der Formel IV können zum Beispiel aus Säurechloriden der Formel $R_2$—alk—COCl und einem Amin der Formel $HN(CH_2—CH_2Hal)_2$ in üblicher Weise erhalten werden. Hal ist vorzugsweise Chlor, Brom oder Jod. Hierzu werden beispielsweise äquimolare Mengen des entsprechenden Säurechlorids, N-(bis-2-halogenethyl)-amin und Triethylamin bei 0°C in Aceton als Lösungsmittel umgesetzt. Man läßt den Ansatz dann noch weitere 8 Stunden bei Raumtemperatur rühren. Danach filtriert man vom entstandenen Triethylaminhydrochlorid ab und engt das Filtrat im Vakuum ein. Der anfallende Rückstand wird ohne weitere Reinigung mit den entsprechenden Anilinen zu den erfindungsgemäßen Verbindungen, wie in Beispiel 39 beschrieben, umgesetzt. Beispielsweise kann das N-(Bis-2-chlorethyl)-3-cyclohexyl-propionsäureamid wie folgt hergestellt werden: 10 ml Aceton werden langsam zu einem auf 0°C abgekühlten Gemisch aus 0,01 Mol (1,8 g) N-(Bis-chlorethyl)-amin-hydrochlorid, 0,02 Mol (1 g) Triethylamin und 0,01 Mol (1,7 g) 3-Cyclohexyl-propionsäurechlorid in 50 ml Aceton zugetropft. Nach beendetem Zutropfen wird weitere 8 Stunden bei Raumtemperatur gerührt. Danach wird vom entstandenen Triethylaminhydrochlorid abfiltriert und das Filtrat im Vakuum eingeengt. Das so erhaltene Produkt wird ohne weitere Reinigung mit 0;03 Mol (1,2 g) 3-Methoxyanilin, wie in Beispiel 39 beschrieben, umgesetzt.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern, wieder in die Basen übergeführt werden. Von den

letzteren lassen sich durch Umsetzung mit organischen oder anorganischen Säuren, insbesondere solchen, die zur Bildung von therapeutisch verwendbaren Salzen geeignet sind, Salze gewinnen. Als solche Säuren seien beispielsweise genannt: Halogenwasserstoffsäuren, Schwefelsäuren, Phosphorsäuren, Salpetersäure, Perchlorsäure, organische Mono-, Di- oder Tricarbonsäuren der aliphatischen, alicyclischen, aromatischen oder heterocyclischen Reihe sowie Sulfonsäuren. Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein- oder Brenztraubensäure; Phenylessig-, Benzoe-, p-Amino-benzoe-, Anthranil-, p-Hydroxy-benzoe-, Salicyl- oder p-Aminosalicylsäure, Embonsäure, Methansulfon-, Äthansulfon-, Hydroxyäthansulfon-, Äthylensulfonsäure; Halogenbenzolsulfon-, Toluolsulfon-, Naphthalinsulfonsäure oder Sulfanilsäure oder auch 8-Chlor-theophyllin.

Diejenigen Verbindungen, die asymmetrische Kohlenstoffatome enthalten und in der Regel als Racemate anfallen, können in an sich bekannter Weise, zum Beispiel mittels einer optisch aktiven Säure, in die optisch aktiven Isomere gespalten werden. Es ist aber auch möglich, von vornherein optisch aktive beziehungsweise auch diastereomere Ausgangsstoffe einzusetzen, wobei dann als Endprodukt eine entsprechende reine optisch aktive Form beziehungsweise diastereomere Konfiguration erhalten wird.

Es können auch diastereomere Racemate auftreten, falls in den hergestellten Verbindungen zwei oder mehr asymmetrische Kohlenstoffatome vorhanden sind. Trennung ist auf üblichem Weg, zum Beispiel durch Umkristallisieren möglich.

Die erfindungsgemäßen Verbindungen sind zur Herstellung pharmazeutischer Zusammensetzungen beziehungsweise Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel kann unter Verwendung der bekannten und üblichen pharmazeutischen Trägermittel und Hilfsstoffe erfolgen.

Die Arzneimittel können beispielsweise enteral, parenteral, oral, perlingual oder in Form von Sprays angewendet werden. Die Verabreichung kann zum Beispiel in Form von Tabletten, Kapseln, Pillen, Dragees, Zäpfchen, Liquida oder Aerosolen erfolgen. Als Liquida kommen zum Beispiel in Frage: ölige oder wässrige Lösungen oder Suspensionen, Emulsionen, injizierbare wässrige oder ölige Lösungen oder Suspensionen.

Pharmakologische beziehungsweise pharmazeutische Angaben

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Enzyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete Cantor KG. Aulendorf in Württemberg 1971.

Beispiele hierfür sind Gelatine, natürliche Zucker wie Rohrzucker oder Milchzucker, Lecithin, Pektin, Stärke (zum Beispiel Maisstärke), Gelatine, Gummiarabikum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Benzylbenzoat, Dioxolane, Glyzerinformale, Tetrahydrofurfurylalkohol, Polyglykolether mit $C_1$—$C_{12}$-Alkoholen, Dimethylacetamid, Lactamide, Lactate, Ethylcarbonate, Silicone (insbesondere mittelviskose Dimethylpolysiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel: Polyacrylsäureester, Celluloseether und ähnliche.

Zur Herstellung von Lösungen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxyd, Fettalkohole, Triglyceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel: 1,3-Butandiol, Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Tragacanth, polyoxyethyliertes Sorbitanmonooleat, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solche die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnussöl, Rhizinusöl, Sesamöl, Baumwollsaatöl, Maisöl (siehe auch Dr. H. P. Fiedler "Lexikon der Hilfsstoffe für Pharmazie, Kosmetik und angrenzende Gebiete" 1971, Seite 191 bis 195).

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich.

Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80°C, vorzugsweise 20 bis 50°C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation der Wirkstoffe beziehungsweise der Arzneimittel kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.

Die erfindungsgemäßen Verbindungen zeigen im Elektroschmerz-Test, Maus (B. Blake et al., Med. exp. 9, 146, 1963) bzw. Haffner-Test, Maus (F. Haffner, Dtsch, Med. Wschr. 55, 731, 1929) eine gute analgetische Wirkung.

Beispielsweise wird bei obengenannten Versuchsmethoden bei einer Dosis von 20 mg/Körpergewicht kg Maus per os die analgetische Wirkung erhalten.

Diese analgetische Wirkung ist mit der Wirkung der bekannten Arzneimittel Pethidin und Pentazocin vergleichbar.

Die niedrigste, bereits deutlich (Wirkung wie oben angegeben) wirksame Dosis in den obengenannten Tierversuchen ist beispielsweise

5 mg/kg oral

0,5 mg/kg intravenös.

Als allgemeiner Dosisbereich für die oben genannten Wirkungen (Tierversuch wie oben) kommt beispielsweise in Frage:

5—100 mg/kg oral, insbesondere 10—40 mg/kg

0,5—10 mg/kg intravenös, insbesondere 0,1—4 mg/kg.

Indikationen, für die die erfindungsgemäßen Verbindungen in Betracht kommen können: Schmerzen verschiedener Ursachen wie postoperative Schmerzen, Wund- und Zahnschmerzen.

Die pharmazeutischen Zubereitungen enthalten im allgemeinen zwischen 0,5 bis 150 mg, vorzugsweise 10 bis 100 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 10 und 50 mg oder Lösungen, die zwischen 1 bis 10% an aktiver Substanz enthalten.

Die Einzeldosis der erfindungsgemäßen aktiven Komponenten kann beispielsweise liegen

a) bei oralen Arzneiformen zwischen 5 bis 100 mg, vorzugsweise 10 bis 50 mg

b) bei parenteralen Arzneiformen (zum Beispiel intravenös, intramuskulär) zwischen 0,5 bis 10 mg, vorzugsweise 1 bis 5 mg

c) bei Arzneiformen zur rektalen oder vaginalen Applikation zwischen 0,5 bis 300 mg, vorzugsweise 10 bis 100 mg.

— (Die Dosen sind jeweils bezogen auf die freie Base) —

Beispielsweise können 3 mal täglich 1 bis 3 Tabletten mit einem Gehalt von 10 bis 50 mg wirksamer Substanz oder zum Beispiel bei intravenöser Injektion 1 bis 5 mal täglich eine Ampulle von 1 bis 10 ml Inhalt mit 1 bis 5 mg Substanz empfohlen werden. Bei oraler Verabreichung ist die minimale tägliche Dosis beispielsweise 30 mg; die maximale tägliche Dosis bei oraler Verabreichung soll nicht über 1 g liegen.

Für die Behandlung von Hunden und Katzen liegt die orale Einzeldosis im allgemeinen zwischen ungefähr 1 und 100 mg/kg Körpergewicht; die parenterale Dosis ungefähr zwischen 0,1 und 10 mg/kg Körpergewicht.

Für die Behandlung von Pferden und Vieh liegt die orale Einzeldosis im allgemeinen zwischen ungefähr 1 und 100 mg/kg; die parenterale Einzeldosis ungefähr zwischen 0,1 und 10 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. *57* (1944) 261) liegt beispielsweise bei oraler Applikation zwischen 100 und 2500 mg/kg (beziehungsweise oberhalb 2500 mg/kg).

Die Arzneimittel können in der Humanmedizin, der Veterinärmedizin sowie in der Landwirtschaft allein oder im Gemisch mit anderen pharmakologisch aktiven Stoffen verwendet werden.

Beispiel 1 (Verfahren a)

1-(3-Methoxy-phenyl)-4-(3-cyclohexyl-propionyl)-piperazin

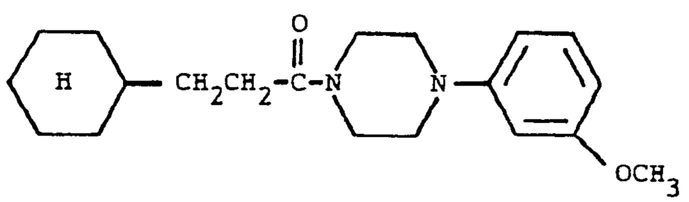

I. Zu einem Gemisch aus 0,06 Mol (11,5 g) N-(3-Methoxyphenyl)-piperazin und 0,06 Mol (6,1 g) Triethylamin in 100 ml absolutem Toluol werden unter Rühren bei Raumtemperatur 0,06 Mol (10,5 g) 3-Cyclohexylpropionsäurechlorid zugetropft. Nach beendetem Zutropfen wird noch 3 Stunden bei Raumtemperatur nachgerührt. Das entstandene Triethylammoniumhydrochlorid wird abfiltriert und das Filtrat im Vakuum eingeengt. Der Rückstand wird in 60 ml Aceton gelöst und tropfenweise mit 11 ml 6n-isopropanolischer Salzsäure versetzt. Das Hydrochlorid fällt aus und wird aus Methylethylketon umkristallisiert.

Ausbeute: 9,9 g

F. des Hydrochlorids 175—176°C.

In einer anderen Ausführung kann wie folgt gearbeitet werden:

II. Ein Gemisch aus 0,05 Mol (7,8 g) 3-Cyclohexylpropionsäure, 0,05 Mol (9,6 g) N-(3-Methoxyphenyl)piperazin und 0,05 Mol (10,3 g) N,N-Dicyclohexylcarbodiimid, gelöst in 150 ml wasserfreiem Methylenchlorid wird 7 Tage bei Raumtemperatur gerührt.

Nach beendeter Reaktion wird vom entstandenen Dicyclohexylharnstoff abfiltriert, das Filtrat im Vakuum eingeengt und das Rohprodukt wie oben angegeben in das Hydrochlorid überführt.

Ausbeute: 3,7 g

F. des Hydrochlorids 175—176°C.

III. Ein Gemisch aus 0,05 Mol (8,5 g) 3-Cyclohexylpropionsäuremethylester, 0,06 Mol (11,5 g) N-(3-Methoxy-phenyl)-piperazin, gelöst in 100 ml Toluol wird 8 Stunden unter Rückfluß erhitzt, wobei der bei der Reaktion gebildete Alkohol (MeOH) abdestilliert wird. Nach beendeter Reaktion wird das Lösungsmittel im Vakuum abgezogen. Das Rohprodukt wird wie beschrieben durch Zusatz von 6n-isopropanolischer HCl in das Hydrochlorid überführt und dieses zur weiteren Reinigung aus Methylethylketon umkristallisiert.

Ausbeute: 4,6 g

F. des Hydrochlorids 175—176°C.

Analog Beispiel 1 (nach Verfahren a) werden die folgenden in Tabelle 1 aufgeführten Verbindungen der Formel I erhalten:

TABELLE 1

| Beispiel | Verfahrensprodukt | | | | | Ausgangsstoffe |
|---|---|---|---|---|---|---|
| | $R_1$ | $R_2$ | alk | Ausbeute in g | F. (0°C) | |
| 2 | 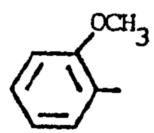 | Cyclohexyl | $(CH_2)_2$ | 12,3 | 144–145 (HCl–Salz) | 11,5 g N-(o-Methoxy-phenyl)-piperazin und 10,5 g 3-Cyclohexyl-propionsäurechlorid |
| 3 | 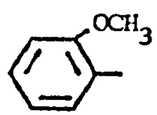 | Cyclohexyl | $CH_2$ | 10,9 | 150–153 (HCl–Salz) | 7,8 g 2-Cyclohexyl-essigsäure und 9,6 g N-(2-Methoxy-phenyl)-piperazin |
| 4 | 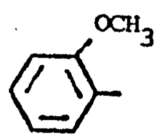 | 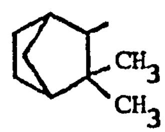 | $(CH_2)_2$ | 9,0 | 198–199 (HCl–Salz) | 11,5 g (2-Methoxy-phenyl)-piperazin und 12,9 g 3-(3,3-Dimethyl-bicyclo[2.2.1]-hept-2-yl)-propionsäurechlorid |
| 5 | 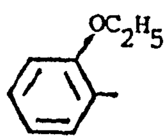 | wie bei Beispiel 4 | $(CH_2)_2$ | 5,3 | 175 (HCl–Salz) | 12,4 g N-(2-Ethoxy-phenyl)-piperazin und 12,9 g 3-(3,3-Dimethyl-bicyclo[2.2.1]-hept-2-yl)-propionsäurechlorid |
| 6 | 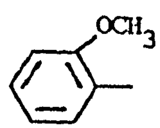 | wie bei Beispiel 4 | $CH_2$ | 11,8 | 183–187 (HCl–Salz) | 11,5 g N-(2-Methoxy-phenyl)-piperazin und 11 g (3,3-Dimethyl-bicyclo[2.2.1]-hept-2-yl)-essigsäurechlorid |

TABELLE 1 (Fortsetzung)

| Beispiel | Verfahrensprodukt | | | | | Ausgangsstoffe |
|---|---|---|---|---|---|---|
| | $R_1$ | $R_2$ | alk | Ausbeute in g | F. (0°C) | |
| 7 | 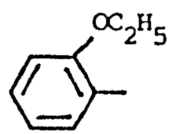 | wie bei Beispiel 4 | $CH_2$ | 10,1 | 107 (Base) | 12,4 g N-(2-Ethoxy-phenyl)-piperazin und 11 g (3,3-Dimethyl)-bicyclo[2.2.1]-hept-2-yl)-essigsäurechlorid |
| 8 | 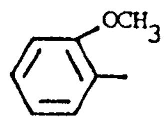 | 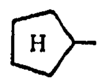 | $(CH_2)_2$ | 4,9 | 155–156 (HCl–Salz) | 9,6 g N-(2-Methoxy-phenyl)-piperazin und 7,1 g 3-Cyclopentyl-propionsäure |
| 9 | 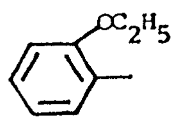 | 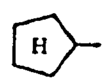 | $(CH_2)_2$ | 4,8 | 134–136 (HCl–Salz) | 10,3 g N-(2-Ethoxy-phenyl)-piperazin und 7,1 g 3-Cyclopentyl-propionsäure |
| 10 | 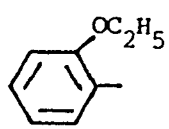 | Cyclohexyl | $(CH_2)_2$ | 12,6 | 147–148 (HCl–Salz) | 12,4 g N-(2-Ethoxy-phenyl)-piperazin und 10,5 g 3-Cyclohexyl-propionsäurechlorid |
| 11 | 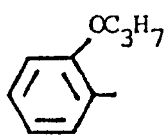 | Cyclohexyl | $(CH_2)_2$ | 9,2 | 146 (HCl–Salz) | 13,2 g N-(2-n-Propyloxy-phenyl)-piperazin und 10,5 g 3-Cyclohexyl-propionsäurechlorid |

TABELLE 1 (Fortsetzung)

| Beispiel | Verfahrensprodukt | | | | | Ausgangsstoffe |
|---|---|---|---|---|---|---|
| | $R_1$ | $R_2$ | alk | Ausbeute in g | F. (0°C) | |
| 12 | 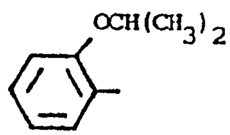 | Cyclohexyl | $(CH_2)_2$ | 5,2 | 59–63 (Base) | 13,2 g N-(2-Isopropyloxyphenyl)-piperazin und 10,5 g 3-Cyclohexyl-propionsäurechlorid |
| 13 | 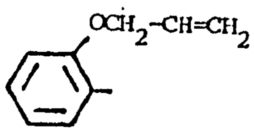 | Cyclohexyl | $(CH_2)_2$ | 8,3 | 37–39 (Base) | 13 g N-(2-Allyloxy-phenyl)-piperazin und 10,5 g 3-Cyclohexyl-propionsäurechlorid |
| 14 | 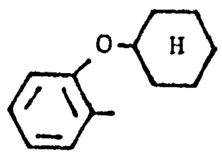 | Cyclohexyl | $(CH_2)_2$ | 5,0 | 70–72 (Base) | 15,6 g N-(2-Cyclohexyloxy-phenyl)-piperazin und 10,5 g 3-Cyclohexyl-propionsäurechlorid |
| 15 | 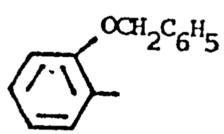 | Cyclohexyl | $(CH_2)_2$ | 11,5 | 86–87 (Base) | 16,8 g N-(2-Benzyloxy-phenyl)-piperazin und 10,5 g 3-Cyclohexyl-propionsäurechlorid |
| 16 | 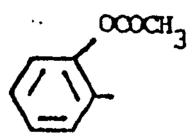 | Cyclohexyl | $(CH_2)_2$ | 5,8 | 61 (Base) | 13,1 g N-(2-Acetoxy-phenyl)-piperazin und 10,5 g 3-Cyclohexyl-propionsäurechlorid |

TABELLE 1 (Fortsetzung)

| Beispiel | Verfahrensprodukt | | | | | Ausgangsstoffe |
|---|---|---|---|---|---|---|
| | $R_1$ | $R_2$ | alk | Ausbeute in g | F. (0°C) | |
| 17 | 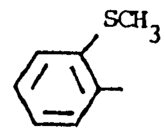 | Cyclohexyl | $(CH_2)_2$ | 8,5 | 111–113 (HCl–Salz) | 10,5 g N-(2-Mercapto-phenyl)-piperazin und 12,5 g 3-Cyclohexyl-propionsäurechlorid |
| 18 | 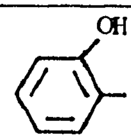 | Cyclohexyl | $(CH_2)_2$ | 6,1 | 128–130 (Base) | 10,7 g N-(2-Hydroxy-phenyl)-piperazin und 10,5 g 3-Cyclohexyl-propionsäurechlorid |
| 19 | 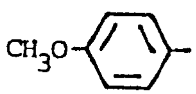 | Cyclohexyl | $(CH_2)_2$ | 15,0 | 173–175 (HCl–Salz) | 7,8 g 3-Cyclohexyl-propionsäure und 9,6 g N-(4-Methoxy-phenyl)-piperazin |
| 20 | 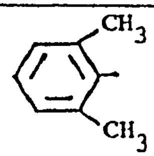 | Cyclohexyl | $(CH_2)_2$ | 6,9 | 73–74 (Base) | 11,4 g N-(2,6-Dimethyl-phenyl)-piperazin und 10,5 g 3-Cyclohexyl-propionsäurechlorid |
| 21 | 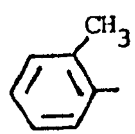 | Cyclohexyl | $(CH_2)_2$ | 8,6 | 158 (HCl–Salz) | 10,5 g N-(2-Methyl-phenyl)-piperazin und 10,5 g 3-Cyclohexyl-propionsäurechlorid |

TABELLE 1 (Fortsetzung)

| Beispiel | Verfahrensprodukt | | | | | Ausgangsstoffe |
|---|---|---|---|---|---|---|
| | $R_1$ | $R_2$ | alk | Ausbeute in g | F. (0° C.) | |
| 22 | 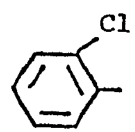 | Cyclohexyl | $(CH_2)_2$ | 6,5 | 114–117 (HCl–Salz) | 11,8 g N-(2-Chlor-phenyl)-piperazin und 10,5 g 3-Cyclohexyl-propionsäurechlorid |
| 23 | 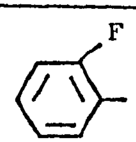 | Cyclohexyl | $(CH_2)_2$ | 11,9 | 134–136 (HCl–Salz) | 10,8 g N-(2-Fluor-phenyl)-piperazin und 10,5 g 3-Cyclohexyl-propionsäurechlorid |
| 24 | 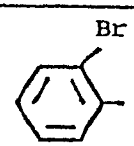 | Cyclohexyl | $(CH_2)_2$ | 7,0 | 52–54 (Base) | 14,5 g N-(2-Brom-phenyl)-piperazin und 10,5 g 3-Cyclohexyl-propionsäurechlorid |
| 25 | 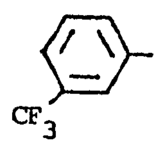 | Cyclohexyl | $(CH_2)_2$ | 7,0 | 160–162 (HCl–Salz) | 13,8 g N-(3-Trifluormethyl-phenyl)-piperazin und 10,5 g 3-Cyclohexyl-propionsäurechlorid |
| 26 | 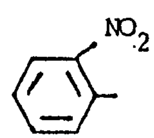 | Cyclohexyl | $(CH_2)_2$ | 15,3 | 61–62 (Base) | 12,4 g N-(2-Nitro-phenyl)-piperazin und 10,5 g 3-Cyclohexyl-propionsäurechlorid |

TABELLE 1 (Fortsetzung)

| Beispiel | Verfahrensprodukt | | | | | Ausgangsstoffe |
|---|---|---|---|---|---|---|
| | $R_1$ | $R_2$ | alk | Ausbeute in g | F. (0°C) | |
| 27 | 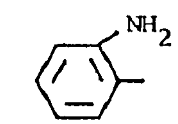 | Cyclohexyl | $(CH_2)_2$ | 4,5 | 202–203 (HCl–Salz) | 8,8 g N-(2-Amino-phenyl)-piperazin und 7,8 g 3-Cyclohexyl-propionsäure |
| 28 | 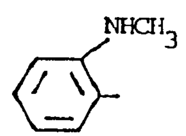 | Cyclohexyl | $(CH_2)_2$ | 6,5 | 89–91 (Base) | 11,5 g N-(2-Methylamino-phenyl)-piperazin und 10,5 g 3-Cyclophenyl-propionsäurechlorid |
| 29 | 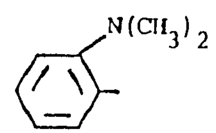 | Cyclohexyl | $(CH_2)_2$ | 4,3 | Öl Rf-Wert 0,72* | 12,2 g N-(2-Dimethylamino-phenyl)-piperazin und 10,5 g 3-Cyclohexyl-propionsäurechlorid |
| 30 | 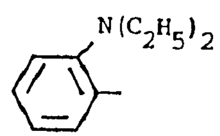 | Cyclohexyl | $(CH_2)_2$ | 8,5 | 77 (Base) | 13,9 g N-(2-Diethylamino-phenyl)-piperazin und 10,5 g 3-Cyclohexyl-propionsäurechlorid |
| 31 | 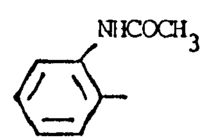 | Cyclohexyl | $(CH_2)_2$ | 6,2 | 107–108 (Base) | 13,1 g N-(2-Acetamino-phenyl)-piperazin und 10,5 g 3-Cyclohexyl-propionsäurechlorid |

* Der Rf-Wert (von englisch retention factor) wurde auf einer Kieselgel-Dunnschichtchromatographieplatte ($Si60_{F254}$ Fa. Merck, Darnstadt) bestimmt. Laufmittel: Chloroform/Methanol/$NH_3$ im Verhältnis 95:4:1.

TABELLE 1 (Fortsetzung)

| Beispiel | Verfahrensprodukt | | | | | Ausgangsstoffe |
|---|---|---|---|---|---|---|
| | $R_1$ | $R_2$ | alk | Ausbeute in g | F. (0°C) | |
| 32 | 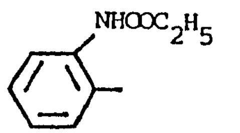 | Cyclohexyl | $(CH_2)_2$ | 6,0 | 93–94 (Base) | 13,9 g N-(2-Propionylamino-phenyl)-piperazin und 10,5 g 3-Cyclohexyl-propionsäurechlorid |
| 33 | 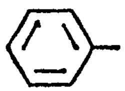 | Cyclohexyl | $(CH_2)_2$ | 9,5 | 175–177 (HCl–Salz) | 9,7 g Phenylpiperazin und 10,5 g 3-Cyclohexyl-propionsäurechlorid |
| 34 | 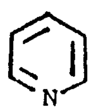 | Cyclohexyl | $(CH_2)_2$ | 9,2 | 89–90 (Base) | 9,8 g N-Pyridyl-(2)-piperazin und 10,5 g 3-Cyclohexyl-propionsäurechlorid |
| 35 | 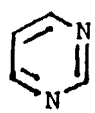 | Cyclohexyl | $(CH_2)_2$ | 7,1 | 104–105 (Base) | 9,8 g N-Pyrimidyl-(2)-piperazin und 10,5 g 3-Cyclohexyl-propionsäurechlorid |
| 36 | 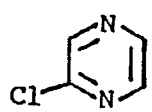 | Cyclohexyl | $(CH_2)_2$ | 10,7 | 95–96 (Base) | 19,8 g N-2-Chlor-pyrazinyl-(6)-piperazin und 10,5 g 3-Cyclohexyl-propionsäurechlorid |

Beispiel 37

1-(2-Methylamino-phenyl)-4-(3-cyclohexyl-propionyl)-piperazin
(die gleiche Verbindung wird nach Beispiel 28 erhalten)

Zu einem Gemisch aus 0,03 Mol (10,4 g) 1-(2-Aminophenyl)-4-(3-cyclohexyl-propionyl)-piperazin und 0,12 Mol (10,1 g) $NaHCO_3$ in 50 ml $H_2O$ werden bei 10°C 0,11 Mol (13,9 g) Dimethylsulfat zugetropft. Nach beendetem Zutropfen läßt man den Ansatz langsam auf Raumtemperatur erwärmen. Danach wird weitere 24 Stunden bei Raumtemperatur gerührt. Anschließend wird 4 Stunden auf 50°C erwärmt. Nach Abkühlen auf Raumtemperatur wird mit 10 %iger NaOH bis zur alkalischen Reaktion versetzt. Es wird dreimal mit je 100 ml Diethylether ausgeschüttelt, die organische Phase abgetrennt, die vereinigten organischen Phasen über $Na_2SO_4$ getrocknet und im Vakuum eingeengt. Der eingeengte Rückstand wird über Kieselgel chromatographiert. Als Laufmittel dient $CHCl_3$/Essigester im Verhältnis 95:5.

Ausbeute: 2,1 g

F. 89—91°C.

Die Alkylierung kann auch mit Methyljodid erfolgen:

Zu einem Gemisch von 0,02 Mol (2,8 g) $K_2CO_3$ und 0,018 Mol (5,5 g) 1-(2-Amino-phenyl)-4-(3-cyclohexylpropionyl)-piperazin, gelöst in 75 ml absoluten Tetrahydrofuran werden unter Rühren 0,02 Mol (2,84 g) Methyljodid, gelöst in 20 ml Tetrahydrofuran, bei Raumtemperatur zugetropft. Der Ansatz wird 7 Tage unter Rückfluß erhitzt. Danach wird vom entstandenen Feststoff abgesaugt, mit $H_2O$ gewaschen, im Vakuum eingeengt und der eingeengte Rückstand an Kieselgel chromatographiert. Als Laufmittel dient ein Gemisch aus Diethylether/Benzin im Verhältnis 1:1.

Ausbeute: 1,6 g

F. 90—91°C.

Beispiel 38

1-(2-Propyloxy-phenyl)-4-(3-cyclohexyl-propionyl)-piperazin
(dieselbe Verbindung wird nach Beispiel 11 erhalten)

0,03 Mol (0,7 g) Natrium werden in 50 ml absolutem Ethanol gelöst. Unter Rühren werden bei Raumtemperatur 0,025 Mol (7,9 g) 1-(2-Hydroxy-phenyl)-4-(3-cyclohexyl-propionyl)-piperazine zugegeben und 1 Stunde unter Rühren am Rückfluß erhitzt. Anschließend wird das Lösungsmittel im Vakuum abgezogen und der Rückstand in 70 ml absolutem Dimethylformamid gelöst. Zu dieser Lösung wird nun eine Lösung von 0,025 Mol (3,1 g) n-Propylbromid in 10 ml Dimethylformamid bei Raumtemperatur zugetropft. Anschließend wird 8 Stunden auf 40—50°C erwärmt. Der Ansatz wird danach im Vakuum eingeengt, der Rückstand mit $H_2O$ versetzt, mit Ammoniak bis zur alkalischen Reaktion versetzt, 3mal mit je 50 ml $CHCl_3$ ausgeschüttelt, die organische Phase über $Na_2SO_4$ getrocknet und im Vakuum eingeengt. Das Rohprodukt wird an Kieselgel chromatographiert. Als Laufmittel dient ein Gemisch aus Essigester/$CHCl_3$ (5:95).

Ausbeute: 2,6 g

F. des Hydrochlorids 146°C.

Werden anstelle von n-Propylbromid 0,025 Mol einer anderen alkylierenden Verbindung verwendet, so erhält man andere Alkylierungsprodukte.

So erhält man gemäß der zuvor angegebenen Arbeitsweise bei Verwendung von 0,025 Mol (3,15 g) Benzylchlorid 5,1 g der Verbindung gemäß Beispiel 15.

F. der Base 86—87°C.

Bei Verwendung von 0,025 Mol (3,02 g) Allylbromid erhält man 5,0 g der Verbindung gemäß Beispiel 13.

F. 37—39°C.

Bei Verwendung von 0,025 Mol (4,07 g) Cyclohexylbromid erhält man 1,8 g der Verbindung gemäß Beispiel 14.

F. 70—72°C.

Beispiel 39

1-(3-Methoxy-phenyl)-4-(3-cyclohexyl-propionyl)-piperazin

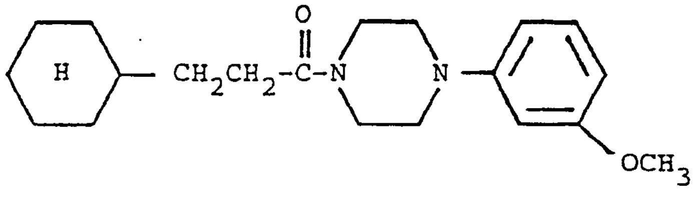

0,01 Mol (2,8 g) N-(Bis-(2-chlorethyl)-3-cyclohexylpropionsäureamid und 0,03 Mol (1,2 g) 3-Methoxyanilin werden in 50 ml Diethylenglykoldimethylether 14 Stunden auf 150°C erhitzt. Die erhaltene Mischung wird mit Wasser versetzt und anschließend 3 mal mit Dichlormethan extrahiert. Die vereinigten Methylen-chlorid-Extrakte werden über $Na_2SO_4$ getrocknet; nach dem Abfiltrieren wird das Filtrat im Vakuum

eingeengt. Der Rückstand wird in 10 ml Aceton aufgenommen und tropfenweise mit 3 ml 6n-isopropanolischer Salzsäure versetzt. Das Hydrochlorid fällt aus und wird aus Methylethylketon umkristallisiert.

Ausbeute: 1,1 g

F. des Hydrochlorids 175—176°C.

Beispiele für pharmazeutische Zubereitungen

Beispiel Kapseln

10 kg der Verbindung gemäß Beispiel 2 (Hydrochlorid) werden in einer Wirbelschicht-Sprühgranulationsapparatur mit einer Lösung von 0,25 kg Gelatine in 2,25 kg Wasser in bekannter Weise granuliert. Nach Zumischen von 0,80 kg Maisstärke, 0,1 kg Magnesiumstearat und 0,05 kg Hochdispersem Siliciumdioxid wird die Mischung in einer Füllmenge von jeweils 224 mg in Hartgelatinekapseln der Größe 1 gefüllt.

Eine Kapsel enthält 200 mg Wirksubstanz.

Beispiel Ampullen

100 g der Verbindung gemäß Beispiel 2 (Hydrochlorid) werden in einer Mischung aus 900 g Propandiol-1,2 und 150 g Ethanol gelöst. Die Lösung wird mit Wasser für Injektionszwecke auf 2 Liter aufgefüllt, über ein Membranfilter geeigneter Porenweite steril filtriert und unter aseptischen Bedingungen zu 2 ml in sterilisierte Ampullen abgefüllt.

Eine Ampulle enthält 100 mg Wirksubstanz in 2 ml Lösung.

**Patentansprüche**

1. Verbindungen der allgemeinen Formel

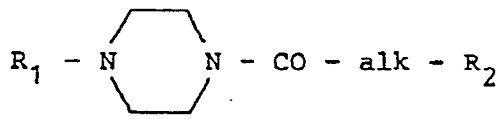

I

worin $R_1$ ein Phenylrest, Pyridylrest, Pyrimidylrest oder Pyrazinylrest oder ein durch die Reste $R_3$ und $R_4$ substituierter Phenylrest, Pyridylrest, Pyrimidylrest oder Pyrazinylrest ist, die Substituenten $R_3$ und $R_4$ gleich oder verschieden sind und Wasserstoff, Fluor, Chlor, Brom, Trifluormethyl, Hydroxyl, $C_1$—$C_6$-Alkylgruppen, $C_1$—$C_6$-Alkoxygruppen, $C_3$—$C_6$-Alkenyloxygruppen, $C_3$—$C_6$-Cycloalkyloxygruppen, Phenyl-$C_1$—$C_4$-alkoxygruppen, $C_1$—$C_6$-Alkylmercaptogruppen, die Nitrogruppe, die Aminogruppe, $C_1$—$C_6$-Alkylaminogruppen, $C_1$—$C_6$-Dialkylaminogruppen, $C_2$—$C_6$-Alkanoylgruppen, $C_2$—$C_6$-Alkanoylaminogruppen oder $C_2$—$C_6$-Alkanoyloxygruppen bedeuten und $R_2$ den Adamantylrest, den 3,3-Dimethylbicyclo[2.2.1]hept-2-yl-rest oder einen gesättigten oder einfach ungesättigten $C_3$—$C_{16}$-Cycloalkylrest darstellt und alk eine gerade oder verzweigte $C_1$—$C_6$-Alkylenkette ist sowie deren physiologisch verträgliche Salze.

2. Verbindungen der Formel I, worin $R_1$ ein Phenylrest oder Pyridylrest oder Pyrimidylrest oder Pyrazinylrest oder Chlorpyrazinylrest oder ein durch die Reste $R_3$ und $R_4$ substituierter Phenylrest ist, wobei $R_3$ Fluor, Chlor, Trifluormethyl, Hydroxyl, $C_1$—$C_6$-Alkyl, $C_1$—$C_6$-Alkoxy, $C_3$—$C_6$-Alkenyloxy, Cyclohexyloxy, Cyclopentyloxy, Phenyl-$C_1$—$C_2$-alkoxy, $C_1$—$C_6$-Alkylmercapto, Nitro, Amino, $C_1$—$C_6$-Alkylamino, $C_1$—$C_6$-Dialkylamino, $C_2$—$C_6$-Alkanoyl, $C_2$—$C_6$-Alkanoylamino oder $C_2$—$C_6$-Alkanoyloxy bedeutet und $R_4$ Wasserstoff oder $C_1$—$C_6$-Alkyl ist, alk aus 1, 2, 3 oder 4 Kohlenstoffatomen besteht und $R_2$ Cycloheptyl oder Cyclohexyl oder Cyclopentyl oder Adamantyl oder 3,3-Dimethylbicyclo[2.2.1]-hept-2-yl ist.

3. Verbindungen der Formel I, worin $R_1$ ein durch die Reste $R_3$ und $R_4$ substituierter Phenylrest ist, wobei $R_3$ Fluor, Chlor, $C_1$—$C_6$-Alkoxy, $C_2$—$C_6$-Alkanoyloxy, $C_2$—$C_6$-Alkanoylamino oder Amino bedeuten und $R_4$ Wasserstoff oder Fluor, Chlor, $C_1$—$C_6$-Alkoxy, $C_2$—$C_6$-Alkanoyloxy, $C_2$—$C_6$-Alkanoylamino oder Amino ist, alk aus 1, 2, 3 oder 4 Kohlenstoffatomen besteht und $R_2$ Cycloheptyl oder Cyclohexyl oder Cyclopentyl oder Adamantyl oder 3,3-Dimethylbicyclo[2.2.1]-hept-2-yl ist.

4. Verbindungen nach einem oder mehreren der vorangegangenen Ansprüche, dadurch gekennzeichnet, daß alk 2, 3 oder 4 C-Atomen enthält.

5. Verbindungen nach einem oder mehreren der vorangegangenen Ansprüche, dadurch gekennzeichnet, daß $R_2$ Cycloheptyl oder Cyclohexyl oder Cyclopentyl oder 3,3-Dimethyl-bicyclo[2.2.1]-hept-2-yl ist.

6. Verbindungen nach einem oder mehreren der vorangegangenen Ansprüche, dadurch gekennzeichnet, daß $R_1$ ein Phenylrest ist, der insbesondere in 2-Stellung durch Fluor, eine $C_1$—$C_6$-Alkoxygruppe, eine $C_2$—$C_6$-Alkanoyloxygruppe oder $C_2$—$C_6$-Alkanoylaminogruppe substituiert ist, die Gruppe alk 2—4 Kohlenstoffatome enthält und $R_2$ einen $C_5$—$C_7$-Cycloalkylreste darstellt.

7. Verfahren zur Herstellung von Verbindungen der allgemeinen Formel

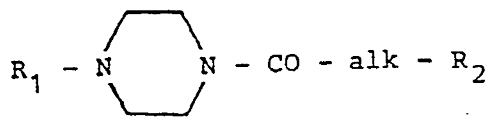

I

worin $R_1$ ein Phenylrest, Pyridylrest, Pyrimidylrest oder Pyrazinylrest oder ein durch die Reste $R_3$ und $R_4$ substituierter Phenylrest, Pyridylrest, Pyrimidylrest oder Pyrazinylrest ist, die Substituenten $R_3$ und $R_4$ gleich oder verschieden sind und Wasserstoff, Fluor, Chlor, Brom, Trifluormethyl, Hydroxyl, $C_1$—$C_6$-Alkylgruppen, $C_1$—$C_6$-Alkoxygruppen, $C_3$—$C_6$-Alkenyloxygruppen, $C_3$—$C_6$-Cycloalkyloxygruppen, Phenyl-$C_1$—$C_4$-alkoxygruppen, $C_1$—$C_6$-Alkylmercaptogruppen, die Nitrogruppe, die Aminogruppe, $C_1$—$C_6$-Alkylaminogruppen, $C_1$—$C_6$-Dialkylaminogruppen, $C_2$—$C_6$-Alkanoylgruppen, $C_2$—$C_6$-Alkanoylaminogruppen oder $C_2$—$C_6$-Alkanoyloxygruppen bedeuten und $R_2$ den Adamantylrest, den 3,3-Dimethylbicyclo[2.2.1]hept-2-yl-rest oder einen gesättigten oder einfach ungesättigten $C_3$—$C_{16}$-Cycloalkylrest darstellt und alk eine gerade oder verzweigte $C_1$—$C_6$-Alkylenkette ist sowie deren physiologisch verträglichen Salzen, dadurch gekennzeichnet, daß man

a) eine Verbindung der allgemeinen Formel

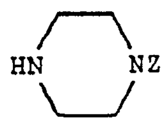

II

worin Z die Gruppe —$R_1$ oder —CO—alk—$R_2$ ist, mit einer Verbindung der allgemeinen Formel

$$Z'X \qquad III$$

umsetzt, worin X ein Halogenatom bedeutet falls Z' die Gruppe $R_1$ und Z die Gruppe —CO—alk—$R_2$ ist oder worin X ein Halogenatom oder die Gruppe —OR ist, falls Z' die Gruppe —CO—alk—$R_2$ und Z die Gruppe $R_1$ ist und R Wasserstoff, einen $C_1$—$C_6$-Alkylrest, einen Benzylrest oder einen gegebenenfalls durch Chlor, Brom, Nitrogruppen oder $C_1$—$C_4$-Alkylgruppen substituierten Phenylrest darstellt, oder

b) eine Verbindung der allgemeinen Formel

$$R_2—alk—CO—N(CH_2—CH_2Hal)_2 \qquad IV$$

worin Hal ein Halogenatom ist mit einer Verbindung der Formel

$$R_1NH_2 \qquad V$$

umsetzt, und in den erhaltenen Verbindungen gegebenenfalls eine Nitrogruppe zur Aminogruppe reduziert und/oder gegebenenfalls die erhaltenen Verbindungen acyliert und/oder alkyliert oder alkenyliert und/oder in die Säureadditionssalze überführt.

8. Arzneimittel, enthaltend eine Verbindung der allgemeinen Formel I nach Anspruch 1 neben üblichen Träger- und/oder Verdünnungs- beziehungsweise Hilfsstoffen.

9. Verfahren zur Herstellung eines Arzneimittels, dadurch gekennzeichnet, daß eine Verbindung der allgemeinen Formel I nach Anspruch 1 mit gebräuchlichen pharmazeutischen Trägerstoffen beziehungsweise Verdünnungsmitteln zu pharmazeutischen Zubereitungen verarbeitet wird.

10. Verwendung von Verbindungen der allgemeinen Formel I nach Anspruch 1 zur Herstellung von Arzneimitteln.

**Claims**

1. Compounds of the general formula

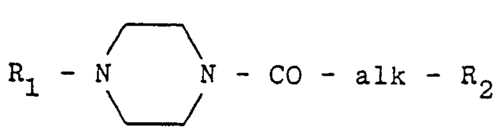

I

wherein $R_1$ represents a phenyl radical, pyridyl radical, pyrimidyl radical or pyrazinyl radical or a phenyl radical, pyridyl radical, pyrimidyl radical or pyrazinyl radical substituted by the radicals $R_3$ and $R_4$, the substituents $R_3$ and $R_4$ are identical or different and represent hydrogen, fluorine, chlorine, bromine, trifluoromethyl, hydroxyl, $C_1$—$C_6$-alkyl groups, $C_1$—$C_6$-alkoxy groups, $C_3$—$C_6$-alkenyloxy groups, $C_3$—$C_6$-cycloalkyloxy groups, phenyl-$C_1$—$C_4$-alkoxy groups, $C_1$—$C_6$-alkylmercapto groups, nitro groups, amino groups, $C_1$—$C_6$-alkylamino groups, $C_1$—$C_6$-dialkylamino groups, $C_2$—$C_6$-alkanoyl groups, $C_2$—$C_6$-alkanoyl-

amino groups or $C_2$—$C_6$-alkanoyloxy groups and $R_2$ represents an adamantyl radical, a 3,3-dimethylbicyclo[2.2.1]hept-2-yl radical or a saturated or simply unsaturated $C_3$—$C_{16}$-cycloalkyl radical and alk represents a straight or branched $C_1$—$C_6$-alkylene chain, as well as physiologically acceptable salts thereof.

2. Compounds of the formula I, wherein $R_1$ represents a phenyl radical or pyridyl radical or pyrimidyl radical or pyrazinyl radical or chloropyrazinyl radical or a phenyl radical substituted by the radicals $R_3$ and $R_4$, where $R_3$ represents fluorine, chlorine, trifluoromethyl, hydroxyl, $C_1$—$C_6$-alkyl, $C_1$—$C_6$-alkoxy, $C_3$—$C_6$-alkenyloxy, cyclohexyloxy, cyclopentyloxy, phenyl-$C_1$—$C_2$-alkoxy, $C_1$—$C_6$-alkylmercapto, nitro, amino, $C_1$—$C_6$-alkylamino, $C_1$—$C_6$-dialkylamino, $C_2$—$C_6$-alkanoyl, $C_2$—$C_6$-alkanoylamino or $C_2$—$C_6$-alkanoyloxy and $R_4$ represents hydrogen or $C_1$—$C_6$-alkyl, alk contains 1, 2, 3 or 4 carbon atoms and $R_2$ represents cycloheptyl or cyclohexyl or cyclopentyl or adamantyl or 3,3-dimethylbicyclo[2.2.1]-hept-2-yl.

3. Compounds of the formula I, wherein $R_1$ represents a phenyl radical substituted by the radicals $R_3$ and $R_4$, where $R_3$ represents fluorine, chlorine, $C_1$—$C_6$-alkoxy, $C_2$—$C_6$-alkanoyloxy, $C_2$—$C_6$-alkanoylamino or amino and $R_4$ represents hydrogen or fluorine, chlorine, $C_1$—$C_6$-alkoxy, $C_2$—$C_6$-alkanoyloxy, $C_2$—$C_6$-alkanoyl amino or amino, alk contains 1, 2, 3 or 4 carbon atoms and $R_2$ represents cycloheptyl or cyclohexyl or cyclopentyl or adamantyl or 3,3-dimethylbicyclo[2.2.1]-hept-2-yl.

4. Compounds according to one or more of the preceding claims, characterised in that, alk contains 2, 3 or 4 carbon atoms.

5. Compounds according to one or more of the preceding claims, characterised in that, $R_2$ represents cycloheptyl or cyclohexyl or cyclopentyl or 3,3-dimethylbicyclo[2.2.1]-hept-2-yl.

6. Compounds according to one or more of the preceding claims, characterised in that, $R_1$ represents a phenyl radical which is substituted, particularly in the 2-position, by fluorine, a $C_1$—$C_6$-alkoxy group, a $C_2$—$C_6$-alkanoyloxy group or $C_2$—$C_6$-alkanoylamino group, the group alk contains from 2 to 4 carbon atoms and $R_2$ represents a $C_5$—$C_7$-cycloalkyl radical.

7. A process for the production of compounds of the general formula

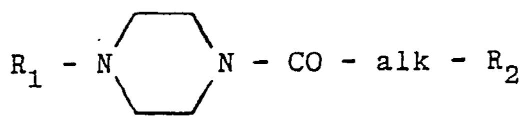

I

wherein $R_1$ represents a phenyl radical, pyridyl radical, pyrimidyl radical or pyrazinyl radical or a phenyl radical, pyridyl radical, pyrimidyl radical or pyrazinyl radical substituted by the radicals $R_3$ and $R_4$, the substituents $R_3$ and $R_4$ are identical or different and represent hydrogen, fluorine, chlorine, bromine, trifluoromethyl, hydroxyl, $C_1$—$C_6$-alkyl groups, $C_1$—$C_6$-alkoxy groups, $C_3$—$C_6$-alkenyloxy groups, $C_3$—$C_6$-cycloalkyloxy groups, phenyl-$C_1$—$C_4$-alkoxy groups, $C_1$—$C_6$-alkylmercapto groups, nitro group, amino group, $C_1$—$C_6$-alkylamino groups, $C_1$—$C_6$-dialkylamino groups, $C_2$—$C_6$-alkanoyl groups, $C_2$—$C_6$-alkanoylamino groups or $C_2$—$C_6$-alkanoyloxy groups and $R_2$ represents the adamantyl radical, the 3,3-dimethylbicyclo[2.2.1]hept-2-yl radical or a saturated or simply unsaturated $C_3$—$C_{16}$-cycloalkyl radical and alk represents a straight or branched $C_1$—$C_6$-alkylene chain, as well as the physiologically acceptable salts thereof, characterised in that,

a) a compound of the general formula

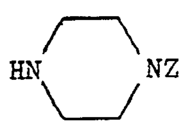

II

wherein Z represents the group —$R_1$ or —CO—alk—$R_2$, is reacted with a compound of the general formula

$$Z'X \qquad III$$

wherein X represents a halogen atom in the case where Z' represents the group $R_1$ and Z represents the group —CO—alk—$R_2$, or X represents a halogen atom or the group —OR, in the case where Z' represents the group —CO—alk—$R_2$ and Z represents the group $R_1$, wherein R represents hydrogen, a $C_1$—$C_6$-alkyl radical, a benzyl radical or a phenyl radical optionally substituted by chlorine, bromine, nitro groups or $C_1$—$C_4$-alkyl groups; or

b) a compound of the general formula

$$R_2\text{—alk—CO—N}(CH_2\text{—}CH_2Hal)_2 \qquad IV$$

wherein Hal represents a halogen atom, is reacted with a compound of the formula

$$R_1NH_2; \qquad V$$

and a nitro group in the compound obtained is optionally reduced to an amino group and/or the compound

obtained is optionally acylated and/or alkylated or alkenylated and/or converted into an acid addition salt.

8. A pharmaceutical composition containing a compound of the general formula I according to claim 1, together with conventional carriers and/diluents or auxiliaries.

9. A process for the production of a pharmaceutical composition characterised in that, a compound of the general formula I according to claim 1 is processed to pharmaceutical preparations with conventional pharmaceutical carriers or diluents.

10. Use of compounds of the general formula I according to claim 1 for the production of pharmaceutical compositions.

**Revendications**

1. Composés de formule générale

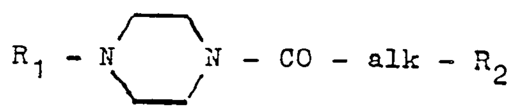

(I)

dans laquelle $R_1$ est un radical phényle, un radical pyridyle, un radical pyrimidyle ou un radical pyrazinyle ou un radical phényle, un radical pyridyle, un radical pyrimidyle ou un radical pyrazinyle substitué par les radicaux $R_3$ et $R_4$, les substituants $R_3$ et $R_4$ sont semblables ou différents et représentent des atomes d'hydrogène, de fluor, de chlore ou de brome, des groupes trifluorométhyle, hydroxyle, alkyle en $C_1$—$C_6$, alcoxy en $C_1$—$C_6$, alcényloxy en $C_3$—$C_6$, cycloalkyloxy en $C_3$—$C_6$, phényl-($C_1$—$C_4$)alcoxy, ($C_1$—$C_6$)alkylmercapto, nitro, amino, ($C_1$—$C_6$)alkylamino, ($C_1$—$C_6$)dialkylamino, alcanoyle en $C_2$—$C_6$, ($C_2$—$C_6$)alcanoylamino ou ($C_2$—$C_6$)alcanoyloxy, $R_2$ représente le radical adamantyle, le radical 3,3-diméthyl-bicyclo[2.2.1]hept-2-yle ou un radical cycloalkyle en $C_3$—$C_{16}$ saturé ou à simple insaturation, et alk est une chaîne alkylène en $C_1$—$C_6$ droite ou ramifiée, ainsi que leurs sels acceptables physiologiquement.

2. Composés de formule I, dans lesquels $R_1$ est un radical phényle, pyridyle, pyrimidyle, pyrazinyle, chloropyrazinyle ou un radical phényle substitué par les radicaux $R_3$ et $R_4$, $R_3$ représentant un atome de fluor ou de chlore, un groupe trifluorométhyle, hydroxyle, alkyle en $C_1$—$C_6$, alcoxy en $C_1$—$C_6$, ($C_3$—$C_6$)alcényloxy, cyclohexyloxy, cyclopentyloxy, phényl-($C_1$—$C_2$)alcoxy, ($C_1$—$C_6$)alkylmercapto, nitro, amino, ($C_1$—$C_6$)alkylamino, ($C_1$—$C_6$)dialkylamino, alcanoyle en $C_2$—$C_6$, ($C_2$—$C_6$)alcanoylamino ou ($C_2$—$C_6$)alcanoyloxy et $R_4$ étant un atome d'hydrogène ou un radical alkyle en $C_1$—$C_6$, alk est formé de 1, 2, 3 ou 4 atomes de carbone et $R_2$ est un radical cycloheptyle, cyclohexyle, cyclopentyle, adamantyle ou 3,3-diméthylbicyclo[2.2.1]-hept-2-yle.

3. Composés de formule I, dans lesquels $R_1$ est un radical phényle substitué par les radicaux $R_3$ et $R_4$, $R_3$ représentant un atome de fluor ou de chlore, un groupe alcoxy en $C_1$—$C_6$, alcanoyloxy en $C_2$—$C_6$, ($C_2$—$C_6$)alcanoylamino ou amino et $R_4$ un atome d'hydrogène, de fluor ou de chlore, un groupe alcoxy en $C_1$—$C_6$, alcanoyloxy en $C_2$—$C_6$, ($C_2$—$C_6$)alcanoylamino ou amino, alk est formé de 1, 2, 3 ou 4 atomes de carbone et $R_2$ est un radical cycloheptyle, cyclohexyle, cyclopentyle, adamantyle ou 3,3-diméthyl-bicyclo[2.2.1]-hept-2-yle.

4. Composés selon l'une quelconque des revendications 1 à 3, caractérisé en ce que alk contient 2, 3 ou 4 atomes de C.

5. Composés selon l'une quelconque des revendications 1 à 4, caractérisé en ce que $R_2$ est un radical cycloheptyle, cyclohexyle, cyclopentyle ou 3,3-diméthyl-bicyclo[2.2.1]-hept-2-yle.

6. Composés selon l'une quelconque des revendications 1 à 4, caractérisé en ce que $R_1$ est un radical phényle qui est substitué, en particulier en position 2, par un atome de fluor, un groupe alcoxy en $C_1$—$C_6$, un groupe alcanoyloxy en $C_2$—$C_6$ ou un groupe ($C_2$—$C_6$)alcanoylamino, le groupe alk contient 2 à 4 atomes de carbone et $R_2$ représente un radical cycloalkyle en $C_5$ à $C_7$.

7. Procédé pour la préparation de composés de formule générale

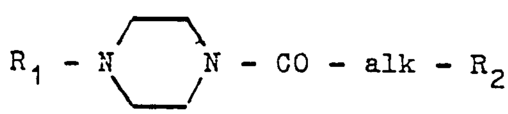

dans laquelle $R_1$ est un radical phényle, pyridyle, pyrimidyle, pyrazinyle ou un radical phényle, pyridyle, pyrimidyle ou pyrazinyle substitué par les radicaux $R_3$ et $R_4$, les substituants $R_3$ et $R_4$ sont semblables ou différents et représentent des atomes d'hydrogène, de fluor, de chlore ou de brome, des groupes trifluorométhyle, hydroxyle, alkyle en $C_1$—$C_6$, alcoxy en $C_1$—$C_6$, alcényloxy en $C_3$—$C_6$, cycloalkyloxy en $C_3$—$C_6$, phényl-($C_1$—$C_4$)alcoxy, ($C_1$—$C_6$)alkylmercapto, nitro, amino, ($C_1$—$C_6$)alkylamino, ($C_1$—$C_6$)dialkylamino, alcanoyle en $C_2$—$C_6$, ($C_2$—$C_6$)alcanoylamino ou alcanoyloxy en $C_2$—$C_6$, $R_2$ représente le radical adamantyle, le radical 3,3-diméthyl-bicyclo[2.2.1]hept-2-yle ou un radical cycloalkyle en $C_3$—$C_{16}$ saturé ou à simple insaturation et alk est une chaîne alkylène en $C_1$—$C_6$ droite ou ramifiée, ainsi que de leurs sels acceptables physiologiquement, caractérisé en ce qu'on fait réagir

a) un composé de formule générale

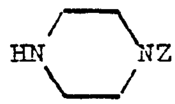

(II)

dans laquelle Z est le groupe $—R_1$ ou $—CO—alk—R_2$, avec un composé de formule générale

Z'X (III)

dans laquelle X représente un atome d'halogène dans le cas où Z' est le groupe $R_1$ et Z est le groupe $—CO—alk—R_2$, et dans laquelle X est un atome d'halogène ou le groupe —OR dans le cas où Z' est le groupe $—CO—alk—R_2$ et Z est le groupe $R_1$, R étant un atome d'hydrogène, un radical alkyle en $C_1—C_6$, un radical benzyle ou un radical phényle éventuellement substitué par des atomes de chlore, de brome, des groupes nitro ou des groupes alkyle en $C_1—C_4$, ou

b) un composé de formule générale

$$R_2—alk—CO—N(CH_2—CH_2Hal)_2 \quad . \qquad (IV)$$

dans laquelle Hal est un atome d'halogène, avec un composé de formule

$$R_1NH_2$$

et, dans les composés obtenus, on réduit éventuellement un groupe nitro en groupe amino et/ou on acyle et/ou on alkyle et/ou on alcényle et/ou on transforme éventuellement en les sels d'acides d'addition les composés obtenus.

8. Médicament contenant un composé de formule générale I selon la revendication 1, outre des excipients et/ou diluants ou substances auxiliaires usuels.

9. Procédé pour la préparation d'un médicament, caractérisé en ce qu'un composé de formule générale I selon la revendication 1 est travaillé avec des excipients ou diluants pharmaceutiques usuels pour former des préparations pharmaceutiques.

10. Utilisation de composés de formule générale I selon la revendication 1 pour la préparation de médicaments.